# EUROPEAN PATENT SPECIFICATION

(11) **EP 3 150 245 B1**
(45) Date of publication and mention of the grant of the patent: **05.09.2018**
(21) Application number: 16191426.2
(22) Date of filing: 29.09.2016
(51) Int. Cl.: A61M 16/04, A61B 1/267, A61M 16/06

(54) **BREATHING MASK COMPRISING AN INTUBATION SYSTEM**
BEATMUNGSMASKE MIT INTUBATIONSVORRICHTUNG
MASQUE DE VENTILATION AVEC UN MÉCANISME D'INTUBATION

(30) Priority: 30.09.2015 QA 90042315
(43) Date of publication of application: 05.04.2017
(73) Proprietor: Chakithandy, Sajith, 3050 Doha (QA)
(72) Inventor: Chakithandy, Sajith, 3050 Doha (QA)
(74) Representative: Monzón de la Flor, Luis Miguel

(56) References cited:
- WO-A1-99/65553
- WO-A1-2004/008951
- WO-A1-2011/060447
- WO-A1-2011/085427
- WO-A1-2012/100016
- WO-A1-2012/172076
- WO-A1-2014/145694
- WO-A2-2011/077407
- CN-Y- 201 175 514
- US-A1- 2011 126 840
- US-A1- 2012 283 513
- US-A1- 2013 197 303
- US-A1- 2013 345 518
- US-A1- 2014 081 084
- US-A1- 2015 119 742
- US-B1- 8 365 734

## Description

### FIELD OF THE INVENTION

The present invention relates to a facemask for ventilation and intubation of an unconscious person and more particularly relates to an anaesthetic mask designed to achieve effective ventilation and simultaneously help secure the airway by passing an endotracheal tube under vision without needing to stop the ventilation.

### BACKGROUND OF THE INVENTION

Mask ventilation and endotracheal intubation are fundamental skills in airway management. The standard masks currently available for a rescuer or anesthetist attempting to perform the basic function of ventilation by using a rigid facemask approximated tightly on the person's face with the rescuers hands. After adequate ventilation is achieved by the facemask an endotracheal tube is passed in to the trachea by removing the facemask and using an instrument called laryngoscope.

A person who has become unconscious from accidental injury or medical reasons or medications administered requires skilled medical personnel to provide the basic function of breathing. There are various procedures and mechanical devices that provide ventilation and protection against airway collapse, air leakage and aspiration for people in these situations. In resuscitation and for minor procedures under anesthesia, a facemask is often sufficient to achieve adequate ventilation. Airway patency of the unconscious patient is maintained either by manipulation of the jaw or by the use of nasopharyngeal or oropharyngeal airway. These are designed to provide a passage for air and oxygen to the windpipe through the nose, mouth and throat of the patient.

Difficult mask ventilation (DMV) is the term used to denote the state where ventilation of an unconscious/anesthetized person is difficult with a facemask. This situation can arise in up to 5% of all anesthesia delivered. DMV develops because of multiple factors that are technique related and/or airway related. Obesity, age older than 55 years, history of snoring, lack of teeth, and the presence of a beard are all independent predictors of DMV. DMV can be even more challenging in infants and children, because they develop low oxygen levels much faster than adults. Thus clinicians should be familiar with the corrective measures and management options when faced with a challenging, difficult, or impossible mask ventilation situation. 'Difficult intubation' is a related situation where passing an endotracheal tube into the patient's trachea is difficult or impossible with direct convention laryngoscopy. Difficulty in achieving tracheal intubation usually occurs because of inability to bring the vocal cords into view through the laryngoscope. This situation needs specialized equipment to solve the problem. The incidence of this situation is about 0.3 to 1.5% in the general population. Fiber optic technology and video laryngoscopy are some of the specialized equipment's developed to assist with 'difficult intubation' situations. Ability to ventilate a patient by viewing his/her vocal cords through the airway and continuing to do so while intubation is being done is an ideal situation in airway management.

Difficult or failures in managing the airway are the major factors underlying morbidity and mortality related to anesthesia. Anesthetic mask described in present invention was developed to facilitate the management of the difficult airway and to reduce the incidence of severe adverse outcomes during airway management.

### PRIOR ART

Reference may be made to US patent 2908269 relating to endotracheal tube holder and bite block assembly for retention of tube but it does not disclose about the ventilation mask with simultaneous intubation.

Yet another reference may be made to US Patent 4270531 relating to oropharyngeal airway tube and bite block assembly having means for connection of a ventilator / resuscitator bag but it does not describe or relate to face mask with airway visualization or relates to ventilation mask with intubation simultaneously as in present invention. Also the technique described here is a blind method with the associated high failure rate due to inadequacy of guess estimation in appropriately positioning an oral airway

Yet another reference may be made to US Patent 5964217 directing towards an endotracheal tube, which can be inserted into a patient's trachea during resuscitation by using a facemask and a curved guide. This invention requires fiber optic equipment for appropriate insertion of the endotracheal tube and is not easily setup. Also the facemask has to be manually held in position to prevent air leak while intubation is being done. There is no guarantee that a clear airway will be maintained before intubation as this is again a blind method. Also secretions may hamper fiber optic usage, which will be an issue in resuscitation situations.

Yet another reference may be made to US Patent 8651102 wherein an emergency ventilation apparatus, system and method for providing ventilation to patients in emergency situation is described. The system described comprises a specially angled laryngoscope handle and a ventilation-aid securement device for securing the facemask and the laryngoscope on the person. This invention does not provide continuous oxygenation and ventilation while endotracheal tube is maneuvered in to place and also it uses a laryngoscope to push the soft tissues away thus having the same failure rate and tissue damage as the currently used laryngoscopic devices.

Reference may be made to another US Patent publication 20140194684 and US Patent publication 20130319406 where a guide to direct the endotracheal tube in to the patient's trachea is described. These guides help with maneuvering the endotracheal tube and also provide oxygen supply near the vocal cords via a flushing channel incorporated in them. As opposed to present invention, these guides are designed to be used as such and not through a facemask as in our invention. Also airway-slider in our invention uses a technique of engaging with the endo tracheal tube and guide it near the vocal cords. Thus maneuverability of the endotracheal tube is much better compared to the above-described guides. The airway slider have a separate channel to keep the patients airway open at all times thus helping with continuous ventilation of the patient at all times.

Reference may be made to another US patent 6860270 (publication no. US2003/0024530) relating to a facemask with two incorporated barrels for ventilation of a patient. The barrels or tubes are inserted blindly in to the nose and oral cavity of the patient. This invention does not relate to maintaining a clear airway of the patient by visualization of the vocal cords. Also it does not help with passing an endotracheal tube under vision in to the patient's windpipe.

Reference may be made to another US patent 4425911 relating to a bite block for use after an endotracheal tube is placed in the patients trachea. This invention does not relate to maintaining the airway and intubating a patient as in our invention, rather it is used for securing the endotracheal tube and for suctioning of oropharynx.

Reference may be made to another US patent 4112936 describing about a bite block and airway assembly comprising a generally U-shaped resilient block and used as a device to maintain a clear airway but no functionality like a self-retaining facemask providing a clear airway by direct visualization of the vocal cords as in present invention

Reference may be made to the US Patent 4848331 wherein apparatus described comprising of oral airways, oral masks and a facemask and combination of these. This apparatus helps with blind maintenance of airway in an unconscious patient and blind passage of endotracheal tube and facilitation of fiber optic assisted intubation. Viewing the windpipe at the same time of ventilating a patient and guiding an endotracheal tube under vision using an airway-slider are unique to present invention.

Reference may be made to another US Patent 8365734 describing multi-port, intubation permitting, oxygen mask. Though the mask is multiport it does not provide or relate to a guide for permitting intubation. Simultaneous ventilation and intubation is not possible with this mask, and only spontaneously breathing patients can have use with the mask described. The mask is used as a standard oxygen mask and is converted to intubation mask when needed. The whole process is done manually thus clinician manually perforates the nasal and/or oral diaphragm to create a point and size of entry perfectly matched to the requirements.

Reference may be made to another US Patent 2013197303A1, where a facemask with an air ventilation port and an intubation port is described. It does not mention a whole sum working apparatus as we described with separate channels designed as bite blocks for endotracheal tube and airway slider incorporating a camera. A guide for endotracheal tube as described in our invention is not described.

Reference may be made to another US patent 20140196726 where in a simple oxygen mask is described having integral bite block and have all necessary opening on the mask for delivering oxygen and help with exhalation. This mask as such was designed for use in endoscopy of a spontaneously breathing patient who is able to actively bite on the bite block and keep the mask in place. The ability of present facemask to create an airtight seal in an unconscious patient, along with ability to introduce medical instruments in to patient's mouth while maintaining the flexibility of removing the facemask without dislodging those medical instruments are not a functionality of this patent.

Reference may be made to US patent no. 5174284 where in endoscopic bite block is described. It has no parts functioning like a facemask as in our invention.

Reference may be made to US2015119742 which, discloses a nasal mask assembly including a nasal mask with a dome shaped body. The dome shaped body has an inner rim and an outer surface. The inner rim has a rounded triangular shape and a tube connector extends from the outer surface. The inner rim is configured such that a bottom portion thereof sits on or above the patient's upper lip while an upper portion thereof extends across the bridge of the patient's nose.

Reference may be made to CN 201175514 a painless gastroscope special mask which comprises a mask main body and a one-way charge valve a Y-shaped tube joint, a mask fixing device, a tooth pad and an attract hole which are arranged on the mask main body.

The entire current airway adjuvants used for difficult mask ventilation are blind techniques, e.g. guedal airway, the size of which is chosen by guess-estimating the distance from teeth to oropharynx. Further there are certain disadvantages of the existing prior arts such as:
- None of the prior art described provide an efficient way of carrying out ventilation and intubation simultaneously and ability to separate the facemask from the endotracheal tube once it is positioned in windpipe;
- Extensive setting up required for management of difficult airway to achieve visualization of the airway, thus less efficient in utilizing time and also patients get very anxious to go through these procedures before they are anaesthetized;
- All the existing techniques or methods are blind techniques and thus will result in higher failure rate and tissue injuries to the patient
- Mask and airway coming together as described in the prior art require one pipe inserted at a time. So it is difficult to maintain airway in-between manipulations and it is different from the present invention, intubation and ventilation can be done simultaneously.

Thus, from the prior arts discussed herein the present invention radically differs from the described references, by providing its specific, functional design, shape & dimensions, hence removing all the aforementioned concerns & dangers for the patients, as described in the statement of the invention and the section on "advantages". The current invention is defined by the features of claim 1. Further features are object of dependent claims 2-10.

### ADVANTAGES OF THE PRESENT INVENTION

Some of the advantages and the object of the unique functional configuration & design of the present invention are listed as follows:
1. One of the advantages of the Ventilation with a view mask is to provide ventilation and intubation simultaneously.
2. The facemask is easy to apply on patient's mouth due to grip of the wings and the channels.
3. Easy and clear view of the vocal cords through camera / prisms lenses and mirrors on airway-slider helps in maintaining a patent airway.
4. Presence of airway-slider with camera / prisms lenses and mirrors helps in correct placement of endotracheal tube.
5. The Ventilation with a view mask is more comfortable to the patient as it needs less manipulation and positioning compared to conventional methods.
6. Minimal manipulation of ventilation mask for intubation helps in reducing tissue damage.
7. Simultaneous intubation and ventilation makes the present invention safe for the patient and cost efficient.
8. Present invention is available in different sizes for all the age group of people i.e. from infants to adults.
9. The whole set of facemask and airway slider incorporating camera / Prisms, lenses and mirrors is made of disposable materials.

### SUMMARY OF THE INVENTION

Accordingly, the present invention seeks to improve airway management in conscious and unconscious patients by a face mask capable of providing ventilation of the patient while allowing intubation of the patient simultaneously, under visual confirmation via a camera / system of lenses, prisms and mirrors. A specially designed airway-slider with an incorporated camera helps with keeping the airway patent and view the vocal cords after being inserted through the above mask.

It is an object of the present invention to provide an anesthetic facemask, which can be used to ventilate and intubate a patient at the same time. It is yet another object of the present invention to provide an airway-slider with camera inserted through the face mask to view the vocal cords for proper ventilation and intubation.

It is yet another object of the present invention to provide two channels attachable to the mask thus providing a passage for airway-slider stick and endotracheal tube while keeping the mouth open.

It is yet another object of the present invention to provide an airtight seal around the channels and lips in such a way that the mask can be separated from the endotracheal tube and airway slider easily without dislodging the endotracheal tube.

It is yet another object of the present invention to provide an airtight seal at the lips and nose by providing 'wings' on the channels and a cushioned base for the nasal part of the facemask.

It is yet another object of the present invention to provide a patent airway in unconscious patients by the airway-slider having a channel in its body.

It is yet another object of the present invention to provide a reusable monitor screen being connected to the airway slider to view the image clearly.

### BRIEF DESCRIPTION OF DRAWINGS

The invention will now be described solely by way of example and with reference to the accompanying drawings in which:
Figure 1: shows a frontal view of an anesthetic face mask according to the present invention
Figure 2: shows a left side perspective view of the mask according to the present invention
Figure 3: shows a cut section view of the mask showing channels.
Figure 4 to 6: shows an Airway-slider with spoon shaped distal end and channel incorporated in the body.

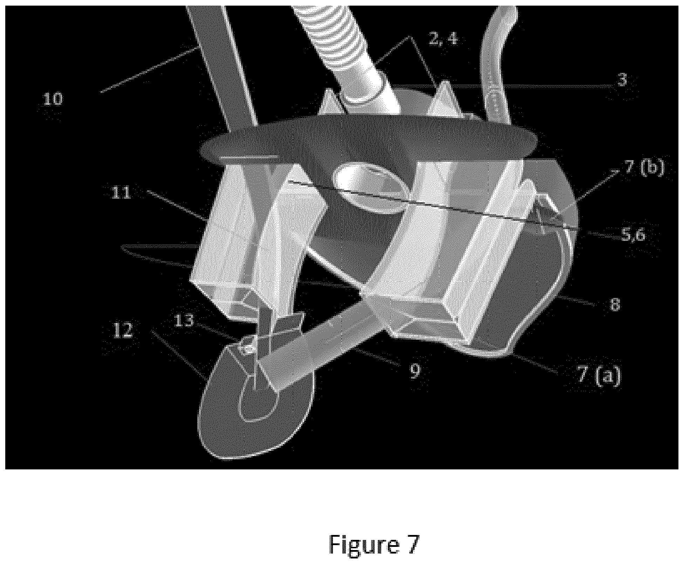

Figure 7: shows an inside view of the mask while inserted in a mouth.

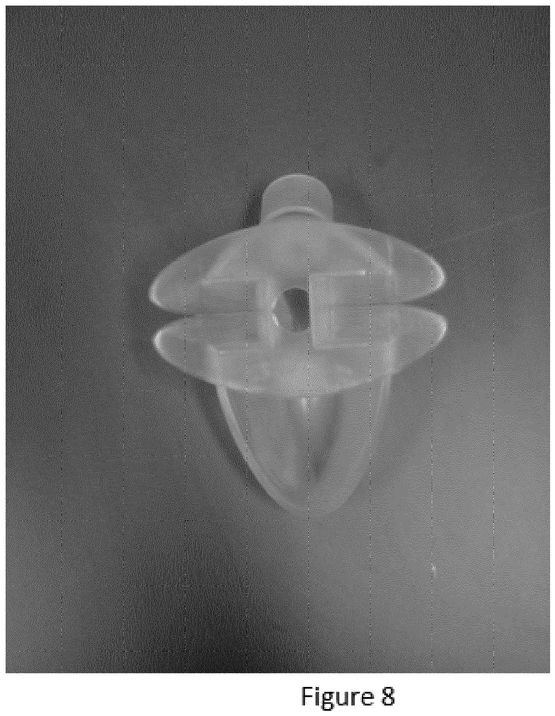

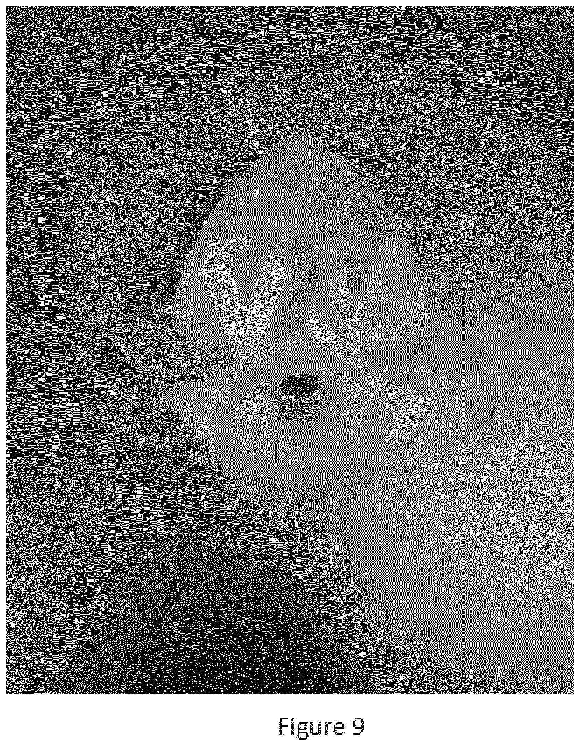

Figure 8 and 9: shows the working prototype of the face mask.

The appended claims particularly point and distinctly claim the subject matter of the present invention. The various objects, advantages and novel features of this invention will be more fully apparent from a reading of the following detailed description in conjunction with the accompanying drawings in which reference numerals refer to like parts.

### DETAILED DESCRIPTION

The facemask of the present invention is designed to be used in routine airway management during anesthesia, intensive care and in emergency medicine but will be particularly useful in 'difficult airway' scenarios in clinical practice by its ability to maintain a clear airway with minimal manipulations by the operator.

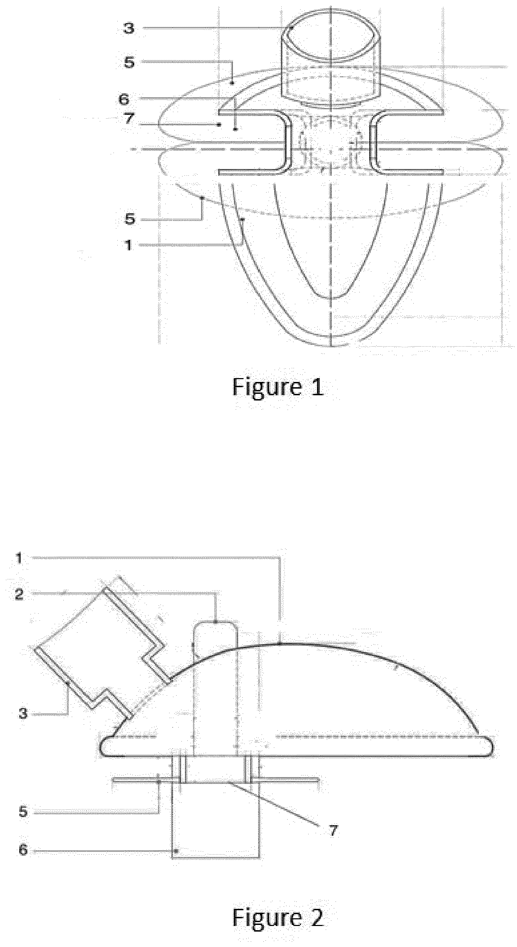

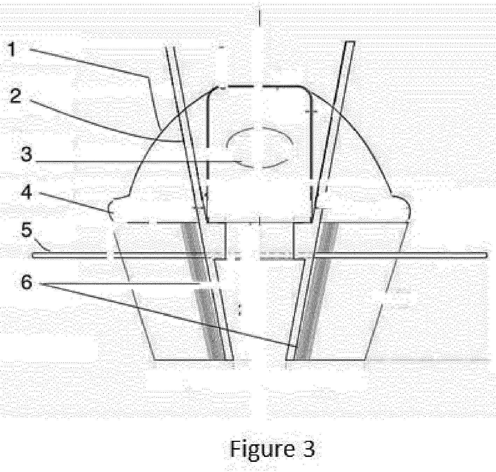

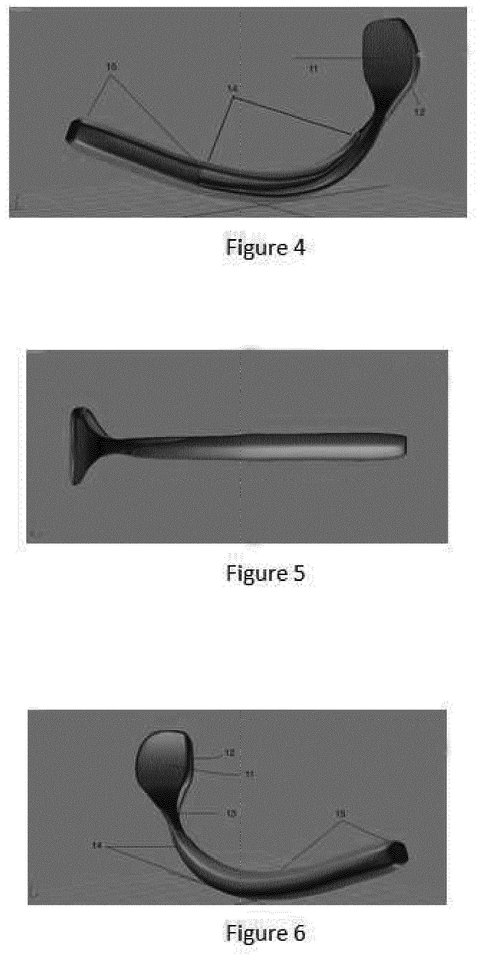

The present invention provides an anesthetic facemask to ventilate and intubate an unconscious person simultaneously and does not require any additional equipment. The ventilation with a view mask (figures 1,2,3) incorporating two channels (6) through one of which 'airway-slider' (figures 4,5,6) fitted with a camera (13) is inserted inside the mouth when the patient is unconscious. The airway-slider simplifies maneuvering an endotracheal tube (9) inserted via the other channel (6) in to the patients' windpipe.

The present invention describes an assembly of face mask (1) with ventilation port (3) and channels (6) with wings (5, 7). The channels are integrated to the lower end of the mask and are inserted in patient's mouth.

More specifically, the present inventions consist of the following parts
Channels (6): help to keep the mouth open when it is inserted inside the mouth. Also it holds the mask in place along with the wings (5,7) attached to it, the wings on the channels when positioned in between the lips and gum/teeth of the patient keep the facemask in position on the patients face, thus freeing operator hands. The channel extends up to the front 1/3rd of the tongue from each side of the ventilation port (3). It is 'C shaped' or 'O shaped' in cross section thus creating space on both sides to insert an airway-slider (figure 4,5,6) on the right side and an endotracheal tube (9) on the left side or vice versa.
Wings on Channels (7(a) and 7(b)): acts as a valve by staying in between the channels (6) and inside of cheeks and on the teeth and gums when inserted properly preventing air leak.
Inside part of the channel (left side/right side): C shaped / O shaped in cross section it extends from front of the mask to reach inside the mouth near the tip of the tongue. It is with or without wings creating an airtight seal between the cheeks and the facemask. One of the channels allows the airway-slider to be inserted through it and engage with the endotracheal tube inserted through the other channel.
Airway-slider (figure 4, 5, 6): this is in effect approximately 30 cm long approximately L shaped to conform with airway anatomy and it has a camera at its slide shaped tip which helps to view inside the mouth after being passed inside the mouth through the right sided channel of the mask. The inner end of the slider is shaped like a spoon with a design feature helping it to engage an endotracheal tube inserted from the left side of the mouth. The camera is positioned a little away from the end giving adequate visualization of the vocal cords. The function of this device is twofold, first to act as an oral airway inside the mouth thus allowing ventilation of the patient with a view of the vocal cords maintained. The second function is to help slide the endotracheal tube in to the wind pipe by minimal maneuvering inside the mouth. The airway -slider may be fixed at any position or angle on to the ventilation mask by use of magnets or velcro thus achieving a clear airway and also guide the endotracheal tube in to patients wind pipe without having to stop ventilation of the patient.
Ventilation port (3): attached at lower end of the mask in line with the nasal opening. It connects to a standard anesthetic circuit or any other breathing circuit. This port is in the center bottom part of the facemask with a clear shoulder part connecting it to the rest of the facemask.
Nasal mask (8): this is the part covering the nose and is attached to the channels at an elevation of 90 degrees this creating an airtight seal.

With specific reference to the drawings in detail, it is stressed that the particulars are shown for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In one embodiment of the present invention a face mask for ventilation and intubation of the patient comprising of a facemask with two channels opening in to the mouth and having wings on the channels to prevent air leak when placed on the patients face and oxygen and anesthetic gases are delivered to the patient through its ventilation port.

In another embodiment an airway-slider for maintaining clear airway and maneuvering an endotracheal tube in to the wind pipe is inserted through the channels on the facemask above and help to keep the airway clear for ventilation by viewing the vocal cords with the camera on it.

In yet another embodiment of the present invention an endotracheal tube is inserted through channel at left and an airway-slider is inserted through channel at right of the mask in to the mouth of a patient. The endotracheal tube is then maneuvered in to the wind pipe with the airway-slider under vision.

In yet another embodiment of the present invention endotracheal tube will be maneuvered through the ventilation port in to patient's nose and guided in to the windpipe with the airway-slider in the throat.

In yet another embodiment of the present invention the airway-slider comprising of three parts:
a) Part 1 (Pharyngeal port) (11): has a thin neck angled about 30 to 50 degree to a spoon shaped end incorporating a camera or system of mirrors, prisms and lenses and has a ridge shaped edge (12) to guide the endotracheal tube
b) Part 2 (body) (14): has a hollow center open on one side and is in a smooth curve corresponding to oral cavity;
c) Part 3 (handle) (15) continuation of the body and it has a wired connection port to be connected to the reusable monitor.

In yet another embodiment of the present invention the Airway-slider is incorporated with a camera at its inner end which provide a video of the inside of the patient on a monitor screen at the bedside

In yet another embodiment of the present invention the face mask is used to clear airway and achieve endotracheal intubation without having to stop ventilation to the patient.

In yet another embodiment of the present invention the face mask is used for all age group of patients by manufacturing them in five different sizes.

In yet another embodiment of the present invention the facemask with incorporated channels will also serve as a soothing dummy to small children needing preoxygenation before getting them to sleep by using a softer material for the channels

In yet another embodiment of the present invention the airway-slider may be designed with a extra channel on it for providing oxygen or using suction near the vocal cords under vision. The ventilator port on the facemask will not be needed in this situation.

In yet another embodiment of the present invention the channels are designed separately from the mask so that they can be used separately or together as needed.

In yet another embodiment of the present invention, the face mask is of 'Q' shaped and hence may be termed as Q-mask.

The facemask is connected to a source of oxygen/air/ anaesthetic vapour, via the ventilation port and the expired gas from the patient is monitored for its contents to achieve adequate oxygenation, ventilation and anaesthesia for the patient.

## Claims

1. A face mask (1) for ventilation and intubation of a patient comprising:
a nasal part (8) for use for ventilating a patient without air leak; a ventilation port (3) attached to the lower end of the mask; an airway-slider (10) incorporating a camera (13); **characterized in that**,
the face mask (1) comprises two channels (6) with or without wings (5,7), which provide access to a patient's mouth and extend inside the mouth near the tip of the tongue, wherein the the two channels (6) are placed on either side of the ventilation port (3) and the camera (13) can in particular be inserted via the channels (6) for the purpose of maintaining the airway by clear visualization of the vocal cords and also to guide an endotracheal tube (9) into a patient's trachea.

2. The face mask as claimed in claim 1 wherein the channels (6) being 'C' or 'O' shaped and the channels (6) and/ or the wings (5,7) being moulded separate from the nasal part (8), such that an operator can attach both of them to the mask with a linking mechanism.

3. The face mask as claimed in claim 1 wherein the channels (6) further have two channel handles (2, 4) for better gripping of the face mask.

4. The face mask as claimed in claim 1 wherein the ventilation port (3) is designed either as a male or female port at the lower end of the nasal part (8) for connection to a standard anaesthetic circuit.

5. The face mask as claimed in any one of the preceding claims wherein the face mask being fabricated of thermoplastic material, silicone or elastomer.

6. The face mask as claimed in any one of the preceding claims wherein said airway-slider further comprising three parts:
a) Pharyngeal part (11) having a thin neck angled about 30-50 degrees relative to a spoon shaped end incorporating a camera (13) or system of mirrors, prisms and lenses and having a ridge shaped edge (12) to guide the endotracheal tube.
b) Body (14) having a hollow center open on one side thus being c-shaped in cross section, designed as a smooth curve corresponding to an oral cavity of the patient;
c) Handle (15) being a continuation of the body (14) and having a wired connection port to be connected to a reusable video monitor and a channel for administering oxygen or suction.

7. The face mask as claimed in claim 6 wherein the airway-slider incorporates the camera (13) at its inner end which provides a video of the inside of the patient's mouth on a monitor screen at the bedside.

8. The face mask as in claim 1 wherein the airway-slider has a system of mirrors, lenses and prisms to transmit the images from inside the patient's mouth to an operator eyepiece on a handle of the airway-slider.

9. The face mask as in claim 2 wherein the airway-slider has a Velcro or magnetic system incorporated on its handle so as to fix it at any specific position onto the 'C' or 'O' shaped channels in the mask.

10. The face mask as claimed in claim 6 wherein the airway-slider is made of a material such as thermoplastic or glass

## Patentansprüche

1. Eine Gesichtsmaske (1) zur Beatmung und Intubation eines Patienten umfassend:
einen Nasenteil (8) zur Verwendung zur Beatmung eines Patienten ohne Luftverlust;
einen Belüftungsanschluss (3), angebracht am unteren Ende der Maske;
einen Atemweg-Schieber (10) mit einer Kamera (13);
**dadurch gekennzeichnet, dass** die Gesichtsmaske (1) zwei Kanäle (6) mit oder ohne Flügel (5, 7) umfasst, die Zugang zum Mund eines Patienten bieten und sich innerhalb des Mundes bis in die Nähe der Zungenspitze erstrecken, wobei die zwei Kanäle (6) jeweils an den Seiten des Beatmungsanschlusses (3) angeordnet sind und die Kamera (13) im Besonderen durch die Kanäle (6) eingesetzt werden kann, um den Atemweg durch eine klare Visualisierung der Stimmbänder aufrecht zu erhalten und auch um einen Endotrachealtubus (9) in die Luftröhre eines Patienten einzuführen.

2. Die Gesichtsmaske nach Anspruch 1, wobei die Kanäle (6) "C"- oder ""O"-förmig sind und die Kanäle (6) und/oder die Flügel (5, 7) vom Nasenteil (8) getrennt geformt sind, sodass ein Bediener beide mit einem Verbindungsmechanismus an der Maske befestigen kann.

3. Die Gesichtsmaske nach Anspruch 1, wobei die Kanäle (6) weiter zwei Kanalgriffe (2, 4) haben, um einen besseren Griff an der Gesichtsmaske sicherzustellen.

4. Die Gesichtsmaske nach Anspruch 1, wobei der Beatmungsanschluss (3) entweder als männlicher oder weiblicher Anschluss am unteren Ende des Nasenteils (8) entworfen ist, um eine Verbindung mit einem standardmäßigen Narkosekreis herzustellen.

5. Die Gesichtsmaske nach einem der vorstehenden Ansprüche, wobei die Gesichtsmaske aus thermoplastischen Material, Silikon oder Elastomer hergestellt ist.

6. Die Gesichtsmaske nach einem der vorstehenden Ansprüche, wobei der erwähnte Atemweg-Schieber weiter drei Teile umfasst:
a) Pharingealer Teil (11) mit einem dünnen Hals, der in einem Winkel von 30-50 Grad im Vergleich zu einem löffelförmigen Ende abgewinkelt ist, mit einer Kamera (13) oder einem System aus Spiegeln, Prismen und Linsen und einer kammförmigen Kante (12) zur Führung des Endotrachealtubus.
b) Körper (14) mit einer hohlen und auf einer Seite offenen Mitte, die dadurch C-förmig im Querschnitt ist und als leichte Kurve entsprechend der Mundhöhle des Patienten entworfen ist;
c) Griff (15), der eine Verlängerung des Körpers (14) ist und einen verdrahteten Anschluss zur Verbindung mit einem wiederverwendbaren Videomonitor und einen Kanal zur Verabreichung von Sauerstoff oder Absaugung hat.

7. Die Gesichtsmaske nach Anspruch 6, wobei der Atemweg-Schieber die Kamera (13) an seinem inneren Ende enthält, die ein Video des Inneren des Mundes des Patienten auf einem Monitorbildschirm am Krankenbett bereitstellt.

8. Die Gesichtsmaske nach Anspruch 1, wobei der Atemweg-Schieber ein System aus Spiegeln, Linsen und Prismen zur Übertragung der Bilder aus dem Inneren des Mundes des Patienten ins Okular des Bedieners auf einem Griff des Atemweg-Schiebers enthält.

9. Die Gesichtsmaske nach Anspruch 2, wobei der Atemweg-Schieber ein Klett- oder Magnetsystem auf seinem Griff eingebaut hat, um ihn in beliebiger spezifischen Position auf den "C"- oder "O"-förmigen Kanälen in der Maske zu befestigen.

10. Die Gesichtsmaske nach Anspruch 6, wobei der Atemweg-Schieber aus einem Material wie beispielsweise Thermokunststoff oder Glas hergestellt ist.

## Revendications

1. Masque facial (1) pour ventilation et intubation d'un patient comprenant :
une partie nasale (8) à utiliser pour ventiler un patient sans fuite d'air ;
un port de ventilation (3) fixé à l'extrémité inférieure du masque ;
un mécanisme d'intubation (10) comportant une caméra (13) ;
**caractérisé en ce que**, le masque facial (1) comporte deux canaux (6) avec ou sans ailes (5, 7) qui donnent accès à la bouche d'un patient et s'étendent à l'intérieur de la bouche près de la pointe de la langue, dans lequel les deux canaux (6) sont placés de chaque côté du port de ventilation (3) et la caméra (13) peut notamment être insérée via les canaux (6) pour conserver la voie aérienne par une visualisation claire des cordes vocales et également pour guider un tube endotrachéal (9) dans la trachée d'un patient.

2. Le masque facial tel que revendiqué dans la revendication 1, dans lequel les canaux (6) étant en forme de « C » ou de « O » et les canaux (6) et/ou les ailes (5, 7) étant moulés séparément de la partie nasale (8), de façon qu'un utilisateur puisse les fixer tous les deux au masque avec un mécanisme de liaison.

3. Le masque facial tel que revendiqué dans la revendication 1, dans lequel les canaux (6) possèdent en outre deux poignées de canal (2, 4) pour une meilleure prise du masque facial.

4. Le masque facial tel que revendiqué dans la revendication 1, dans lequel le port de ventilation (3) est conçu soit comme un port mâle, soit comme un port femelle à l'extrémité inférieure de la partie nasale (8) pour raccordement à un circuit d'anesthésie standard.

5. Le masque facial tel que revendiqué dans l'une quelconque des revendications précédentes, où le masque facial est fabriqué en matière thermoplastique, en silicone ou en élastomère.

6. Le masque facial tel que revendiqué dans l'une quelconque des revendications précédentes, dans lequel ledit mécanisme d'intubation comprend en outre trois parties :
a) Partie pharyngienne (11) possédant un fin col coudé à environ 30-50 degrés par rapport à une extrémité en forme de cuillère comprenant une caméra (13) ou un système de miroirs, prismes et lentilles et possédant un bord en forme de crête (12) pour guider le tube endotrachéal.
b) Corps (14) possédant un centre creux ouvert d'un côté qui est donc en forme de C en coupe transversale, conçu comme une courbe lisse correspondant à une cavité orale du patient ;
c) Poignée (15) étant un prolongement du corps (14) et possédant un port de connexion filaire à connecter à un moniteur vidéo réutilisable et un canal pour administration d'oxygène ou aspiration.

7. Le masque facial tel que revendiqué dans la revendication 6, dans lequel le mécanisme d'intubation comprend la caméra (13) à son extrémité interne qui fournit une vidéo de l'intérieur de la bouche du patient sur un écran de moniteur placé au chevet du patient.

8. Le masque facial tel que revendiqué dans la revendication 1, dans lequel le mécanisme d'intubation possède un système de miroirs, lentilles et prismes pour transmettre les images de l'intérieur de la bouche du patient à un oculaire d'opérateur sur une poignée du mécanisme d'intubation.

9. Le masque facial tel que revendiqué dans la revendication 2, dans lequel le mécanisme d'intubation possède un système Velcro ou magnétique intégré à sa poignée de façon à la fixer à n'importe quelle position spécifique sur les canaux en forme de « C » ou de « O » dans le masque.

10. Le masque facial tel que revendiqué dans la revendication 6, dans lequel le mécanisme d'intubation est fabriqué dans un matériau tel que le thermoplastique ou le verre.
